# EUROPÄISCHE PATENTSCHRIFT

(11) **EP 0 621 023 B1**
(45) Veröffentlichungstag und Bekanntmachung des Hinweises auf die Patenterteilung: **02.06.1999**
(21) Anmeldenummer: 94103978.6
(22) Anmeldetag: 15.03.1994
(51) Int. Cl.: A61F 13/06

(54) **Selbstklebende Fertigbandage**
Adhesive bandage
Bandage adhésif

(30) Priorität: 19.04.1993 DE 4312655
(43) Veröffentlichungstag der Anmeldung: 26.10.1994
(73) Patentinhaber: Beiersdorf Aktiengesellschaft, 20245 Hamburg (DE)
(72) Erfinder: Bodenschatz, Stefan, Dr., D-21614 Buxtehude (DE); Staudinger, Peter, D-25439 Tornesch (DE)

(56) Entgegenhaltungen:
- WO-A-80/01758
- DE-A- 4 014 728
- DE-C- 647 580
- US-A- 4 962 768

## Beschreibung

Die Erfindung betrifft eine einseitig selbstklebend beschichtete Fertigbandage zur Stützung des Sprunggelenks.

Die funktionelle Verbandstechnik, das sog. Taping, ist eine Behandlungsmethode zur Prophylaxe und Therapie von Verletzungen, Krankheiten und Veränderungen am Bewegungsapparat. Taping hat zum Ziel, die Kapsel-Band-Strukturen durch gezielt angelegte Zügel nachzubilden und dadurch eine selektive Unterstützung zu erreichen. Der Tapeverband wird dabei streifenweise aus vorzugsweise unelastischen selbstklebenden Bändern angelegt und schützt, stützt und entlastet gefährdete, geschädigte oder gestörte Anteile einer Funktionseinheit. Er erlaubt die funktionelle Belastung im schmerzfreien Bewegungsraum, verhindert aber extreme Bewegungen.

Das Anlegen derartiger Verbände erfordert jedoch fachmännisches Können und Erfahrung. Tape-Verbände können deshalb in der Regel nicht von einem Laien angelegt werden.

Aufgabe der Erfindung war es deshalb, eine Fertigbandage zur Verfügung zu stellen, die zur prophylaktischen Stützung der am häufigsten betroffenen Kapsel-Band-Strukturen des Sprunggelenks geeignet ist und auf Grund ihrer Form auch vom Verwender in einfacher Weise angelegt werden kann.

Gelöst wird diese Aufgabe durch eine auf einer Seite selbstklebend beschichteten Fertigbandage zur Stützung des Sprunggelenks mit zwei länglichen Streifen, die dadurch gekennzeichnet ist, daß der zweite Streifen (2) neben dem ersten Streifen (1) angeordnet und mittig über ein Verbindungsteil (7) mit diesem verbunden ist, wobei der erste Streifen (1) etwa 50 - 60 cm und der zweite Streifen (2) etwa 80 - 100 cm lang sind, der erste Streifen etwa 5 - 6 cm und der zweite Streifen etwa 3,5 - 4,5 cm breit sind und die beiden Streifen in einem Winkel (α) von 0 - 80° rechts und links von dem Verbindungsteil (7) zueinander verlaufen.

Insbesondere gelöst wird diese Aufgabe durch die Verwendung einer auf einer Seite durch eine auf einer Seite selbstklebend beschichtete Fertigbandage zur Stützung des Sprunggelenks mit zwei länglichen Streifen, die dadurch gekennzeichnet ist, daß der erste Streifen (1) derart ausgebildet ist, daß er unter dem Fußgewölbe so angeklebt werden kann, daß jedes seiner Enden sich bis über den Knöchelbereich erstreckt und diesen bedeckt, und der zweite Streifen (2) neben dem ersten Streifen (1) angeordnet und mittig über ein Verbindungsteil (7) mit diesem verbunden ist, und dieser zweite Streifen (2) eine solche Länge aufweist, daß sein eines freies Teilstück (5) von medial kommend über den dorsalen Fußrücken zum Außenknöchel bis zur Ferse und gegebenenfalls weiter bis etwa zum großen Zehen geführt werden kann, und sein anderes freies Teilstück (6) von lateral kommend über den dorsalen Fußrücken zum Innenknöchel bis zur Ferse und gegebenenfalls weiter unterhalb des Außenknöchels bis zur Kleinzehe geführt werden kann.

Ausgegangen wird dabei von einer Bandage gemäß DE-PS 39 31 550, die eine mit einer klebenden Rückseite versehenen Bandage zur Abstützung des Knöchels betrifft und aus zwei länglichen Streifen besteht, die in L-Form miteinander verbunden sind. Von den beiden Streifen wird der eine unter dem Fußgewölbe des Trägers so angeklebt, daß jedes seiner Enden sich bis zum Knöchelbereich erstreckt und diesen bedeckt. Der andere Streifen wird rund um den Knöchel geklebt. Eine derartige Anordnung vermag zwar den Knöchel zu stützen, jedoch nicht das ganze Fußgelenk.

Die erfindungsgemäße Bandage kann dagegen auf Grund der andersgearteten Anordnung des zweiten Streifens und dessen anderer Führung am Fuß das Sprunggelenk wirkungsvoll stützen.

Die Winkel (α) zwischen den beiden Streifen rechts und links von dem Verbindungsteil (7), das praktisch die Mittelachse der Bandage darstellt, kann einen weiten Bereich umfassen von 0 bis etwa 80°. D.h., in dem einen äußersten Fall verlaufen die Streifen parallel zueinander und es besteht nur ein Einschnitt zwischen ihnen, im anderen äußersten Fall stehen sie beinahe rechtwinklich aufeinander. Vorzugsweise verlaufen sie parallel zueinander, aber auch ein Winkel von 25 - 35° hat sich als günstig erwiesen und gibt eine geeignete Führungsrichtung für die Bänder vor.

Um ein Einreißen am inneren Ende der Einschnitte zu verhindern, können sich dort kleine Ausnehmungen (Löcher) (8) und (9) befinden.

Das Verbindungsteil (7) zwischen den beiden Streifen ist vorteilhafterweise so breit wie der Mittelfuß, da es beim Anlegen der Bandage unter diesen zu liegen kommt.

Die Länge der Streifen ist durch deren Anlegetechnik, die weiter unten noch genauer beschrieben wird, vorgegeben. Sie beträgt bei dem ersten Streifen etwa 50 - 60 cm und bei dem zweiten Streifen etwa 80 - 100 cm.

Die Breite der Streifen entspricht etwa der Breite der Klebebänder, die üblicherweise zum Tapen verwendet werden, wobei der kürzere erste Streifen vorzugsweise etwas breiter ausgestaltet ist als der längere zweite Streifen. Ihre Breite beträgt etwa 5 - 6 cm bzw. 3,5 - 4,5, meist 3,75, cm.

Die Bandage ist insgesamt einstückig ausgebildet, wobei sie entweder als Ganzes aus einem großflächigen Bandagenmaterial ausgeschnitten oder ausgestanzt oder aus Einzelteilen zusammengefügt werden kann. Letzteres beispielsweise in der Weise, daß jeweils eine Hälfte des längeren zweiten Streifens (2) mittig an dem kürzeren Streifen (1) durch Nähen, Schweißen oder Kleben befestigt wird.

Die Streifen bestehen vorzugsweise aus unelastischem Material, d.h. in erster Linie aus einem Baumwollgewebe, wie die üblichen Tape-Klebebänder. Sie können jedoch auch ganz oder teilweise aus einem der anderen für Binden und Bandagen bekannten Materialien aus natürlichen oder synthetischen Grundstoffen - auch Polyestergewebe - bestehen und gegebenenfalls eine gewisse Elastizität in Längs- und/oder Querrichtung, insbesondere in Querrichtung, aufweisen. Wichtig dabei ist, daß das Material zwar einerseits genügend Festigkeit aufweist, um dem Gelenk einen guten Halt zu bieten, andererseits aber auch anschmiegsam und anmodellierbar ist.

Auf ihrer der Haut zugewandten Seite ist die Bandage mit einer der bekannten gut haftenden Selbstklebemassen beschichtet auf Basis von Kautschuk oder synthetischen Polymeren. Vorzugsweise sollten diese luft- und wasserdampfdurchlässig sowie gut hautverträglich sein.

Bis zum Gebrauch der Bandage ist diese Klebeschicht mit einem klebstoffabweisend ausgerüsteten Blattmaterial, wie beispielsweise silikonisiertem Papier, abgedeckt.

Es hat sich dabei als günstig erwiesen, diese Abdeckung mehrteilig, beispielsweise dreiteilig auszubilden. Dabei deckt ein Teil, ebenfalls streifenförmig, den ersten kürzeren Streifen der Bandage und je ein weiteres streifenförmiges Teil den längeren Streifen je zur Hälfte ab. Die Abdeckungsteile können als Anbringungshilfe farbig markiert oder nummeriert sein.

Insbesondere ist eine fünfteilige Abdeckung vorteilhaft. Dabei deckt ein Teil den durchgehenden Mittelabschnitt (7) der Bandage ab und vier weitere Streifen je die Teilstücke (3), (4), (5) und (6) der Streifen (1) und (2). Eine nach innen gerundete Kontur (10, 11) an der Grenzlinie zwischen den Abdeckungsteilen kann ihr Abziehen erleichtern.

Das Anlegen der Bandage erfolgt in der Weise, daß zuerst das Trennpapier von dem Mittelabschnitt (7) entfernt und dieser, mit der vorderen Kante etwa in der Mitte des Fußes, an der Fußsohle (plantar) fixiert wird. Dann werden die Trennpapierstreifen von den Teilstücken (3) und (4) des kürzeren, ersten Streifens (1) entfernt und diese jeweils an den Knöcheln hochgeklebt. Die beiden Teilstücke (3), (4) dieses sog. U-Zügels verlaufen über Innen- und Außenknöchel (die Malleolen) zum proximalen Unterschenkel aus und werden an den Sprunggelenkskonturen anmodelliert. Beim Anlegen ist darauf zu achten, daß der Fuß zum Unterschenkel in einem rechten Winkel steht.

Dann wird die Abdeckung von der einen Hälfte (5) des längeren Streifens entfernt und dieser von medial (innenseitig) kommend über den dorsalen Fußrücken zum Außenknöchel und bis zur Ferse hin geklebt. Der noch freie Rest des Streifenteils kann abgeschnitten oder gegebenenfalls weiterführend bis zur großen Zehe angebracht werden. Dieser Teil der Bandage dient insbesondere zur Führung des Ligamentum Kalkaneo Fibulare.

Abschließend wird die Abdeckung von der anderen Hälfte (6) des längeren Streifens entfernt und dieser von lateral kommend über den dorsalen Fußrücken nach medial zum Innenknöchel und bis zur Ferse hin geklebt, die er etwa am Achillessehnenansatz umfaßt. Von dort wird der Streifen weiter unterhalb des Außenknöchels zur Kleinzehe hin geführt und angeklebt. Sein Ende wird unter leichtem Anheben des Fußaußenrandes dorso-plantar fixiert. Dieser Teil der Bandage dient zur unterstützenden Führung des Ligamentum Fibula talare Anterior.

Insgesamt wird durch die Anordnung der Bandage eine wirksame gute Stützung des Sprunggelenks erreicht, welche eine freie Beweglichkeit im schmerzfreien Bewegungsraum zuläßt jedoch endgradig einschränkt und fixiert.

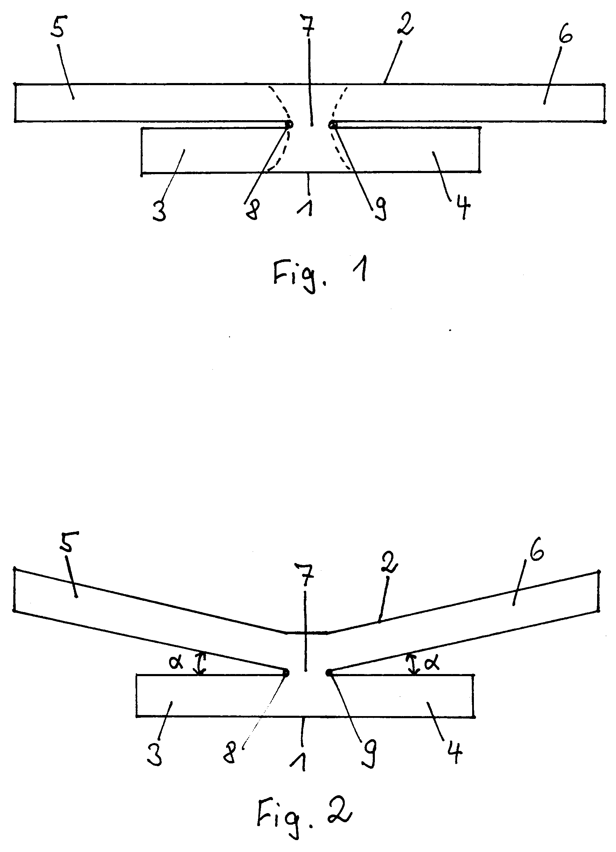

Die erfindungsgemäße Bandage ist in Figur 1 und 2 bespielsweise dargestellt. Dabei bedeuten (1) den ersten Streifen, (2) den zweiten Streifen, (3) und (4) die freien Teilstücke des Streifens (1) sowie (5) und (6) die beiden freien Teilstücke des Streifens (2). (7) bedeutet das Verbindungsteil zwischen den beiden Streifen (1) und (2), (8) und (9) jeweils eine kleine Ausnehmung am inneren Ende der Einschnitte, (10) und (11) die gerundete Kontur der Trennpapierteilstücke und (α) der Winkel zwischen den Streifen (1) und (2).

## Patentansprüche

1. Auf einer Seite selbstklebend beschichtete Fertigbandage zur Stützung des Sprunggelenks mit zwei länglichen Streifen, dadurch gekennzeichnet, daß der zweite Streifen (2) neben dem ersten Streifen (1) angeordnet und mittig über ein Verbindungsteil (7) mit diesem verbunden ist, wobei
der erste Streifen (1) etwa 50 - 60 cm und der zweite Streifen (2) etwa 80 - 100 cm lang sind,
der erste Streifen etwa 5 - 6 cm und der zweite Streifen etwa 3,5 - 4,5 cm breit sind und
die beiden Streifen in einem Winkel (α) von 0 - 80° rechts und links von dem Verbindungsteil (7) zueinander verlaufen.

2. Fertigbandage gemäß Anspruch 1, dadurch gekennzeichnet, daß die beiden Streifen parallel zueinander verlaufen.

3. Fertigbandage gemäß Anspruch 1, dadurch gekennzeichnet, daß die Bandage insgesamt einstückig ausgebildet ist.

4. Fertigbandage gemäß Anspruch 1, dadurch gekennzeichnet, daß die Verbindung zwischen den beiden Streifen durch Kleben, Schweißen oder Nähen hergestellt wurde.

5. Fertigbandage gemäß Anspruch 1, dadurch gekennzeichnet, daß die beiden Streifen auf ihrer klebstoffbeschichteten Seite mit Trennpapier abgedeckt sind.

6. Fertigbandage gemäß Anspruch 5, dadurch gekennzeichnet, daß das Trennpapier dreiteilig ausgebildet ist, wobei ein Teil den ersten Streifen (1) abdeckt und je ein weiteres Teil den zweiten Streifen (2) je zur Hälfte abdeckt.

7. Fertigbandage gemäß Anspruch 5, dadurch gekennzeichnet, daß das Trennpapier fünfteilig ausgebildet ist, wobei ein Teil das Verbindugsteil (7) abdeckt und vier weitere Streifen je ein Teilstück (3), (4), (5) und (6) der Streifen (1) und (2) abdecken.

## Claims

1. Prefabricated bandage which is self-adhesively coated on one side for supporting the ankle joint with two elongated strips, characterized in that the second strip (2) is arranged adjacent to the first strip (1) and is connected centrally thereto via a connection part (7), the first strip (1) being approximately 50 - 60 cm long and the second strip (2) being approximately 80 - 100 cm long, the first strip being approximately 5 - 6 cm wide and the second strip being approximately 3.5 - 4.5 cm wide, and the two strips extending at an angle (α) from 0 - 80° to one another, to the right and left of the connection part (7).

2. Prefabricated bandage according to Claim 1, characterized in that the two strips extend parallel to one another.

3. Prefabricated bandage according to Claim 1, characterized in that the bandage overall is designed in one piece.

4. Prefabricated bandage according to Claim 1, characterised in that the connection between the two strips has been produced by adhesive bonding, welding or sewing.

5. Prefabricated bandage according to Claim 1, characterized in that the two strips are covered on their adhesive-coated side with release paper.

6. Prefabricated bandage according to Claim 5, characterized in that the release paper is designed in three parts, one part covering the first strip (1) and one further part in each case covering each half of the second strip (2).

7. Prefabricated bandage according to Claim 5, characterized in that the release paper is designed in five parts, one part covering the connection part (7) and four further strips each covering a part (3), (4), (5) and (6) of the strips (1) and (2).

## Revendications

1. Bandage préfabriqué enduit d'un côté de manière auto-adhésive pour supporter l'articulation tibiotarsienne avec deux bandes longitudinales, caractérisé en ce que la deuxième bande (2) est disposée près de la première bande (1) et est raccordée à celle-ci au milieu par un morceau de raccordement (7), la première bande (1) mesurant environ 50 - 60 cm de long et la deuxième bande (2) environ 80 - 100 cm de long, la première bande mesurant environ 5 - 6 cm de large et la deuxième bande (2) environ 3,5 - 4,5 cm de large et les deux bandes s'étendant suivant un angle (α) de 0 - 80° l'une par rapport à l'autre à droite et à gauche du morceau de raccordement (7).

2. Bandage préfabriqué selon la revendication 1, caractérisé en ce que les deux bandes s'étendent parallèlement l'une à l'autre.

3. Bandage préfabriqué selon la revendication 1, caractérisé en ce que le bandage est formé dans l'ensemble d'une seule pièce.

4. Bandage préfabriqué selon la revendication 1, caractérisé en ce que le raccordement entre les deux bandes a été effectué par collage, soudure ou couture.

5. Bandage préfabriqué selon la revendication 1, caractérisé en ce que les deux bandes sont recouvertes sur leur côté enduit d'adhésif avec du papier de séparation.

6. Bandage préfabriqué selon la revendication 5, caractérisé en ce que le papier de séparation est formé en trois parties, une partie recouvrant la première bande (1) et une autre partie recouvrant à chaque fois la deuxième bande (2) jusqu'à sa moitié.

7. Bandage préfabriqué selon la revendication 5, caractérisé en ce que le papier de séparation est formé en cinq parties, une partie recouvrant le morceau de raccordement (7) et quatre autres bandes recouvrant respectivement les morceaux (3), (4), (5) et (6) des bandes (1) et (2).
